(19) Europäisches Patentamt
European Patent Office
Office européen des brevets

(11) Veröffentlichungsnummer: **0 233 602 B1**

# (12) EUROPÄISCHE PATENTSCHRIFT

(45) Veröffentlichungstag der Patentschrift: **29.09.93**

(21) Anmeldenummer: **87102008.7**

(22) Anmeldetag: **13.02.87**

(51) Int. Cl.5: **C07C 69/75**, C07C 69/76, C07C 69/90, C07D 317/24, C07D 307/20, C09K 19/58, G09F 9/35

## (54) Chirale Umsetzungsprodukte aus mesogenen Molekülbausteinen und bifunktionell reaktionsfähigen Butantetraolderivaten und ihre Verwendung als Dotierstoff in Flüssigkristall-Phasen.

(30) Priorität: **17.02.86 DE 3604899**

(43) Veröffentlichungstag der Anmeldung: **26.08.87 Patentblatt 87/35**

(45) Bekanntmachung des Hinweises auf die Patenterteilung: **29.09.93 Patentblatt 93/39**

(84) Benannte Vertragsstaaten: **DE**

(56) Entgegenhaltungen:
**DE-A- 3 333 677**
**GB-A- 2 122 198**
**US-A- 4 264 148**

(73) Patentinhaber: **HOECHST AKTIENGESELLSCHAFT**

**D-65926 Frankturt(DE)**

(72) Erfinder: **Scherowsky, Günter, Prof. Dr.**
**Winklerstrasse 18B**
**D-1000 Berlin 33(DE)**
Erfinder: **Gunaratne, Manel**
**Fraunhoferstrasse 26**
**D-1000 Berlin 10(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Kennlinien der in Flüssigkristall-Displays verwendeten elektro-optischen Effekte verändern sich im allgemeinen mit der Temperatur. Insbesondere bei einer Ansteuerung im Multiplexbetrieb ergeben sich daraus Schwierigkeiten die zu einer unerwünschten Einschränkung des Arbeitstemperaturbereiches führen können. Bei verschiedenen elektrooptischen Effekten kann durch Zusatz chiraler Verbindungen zum nematischen Flüssigkristall über die Temperaturfunktion der Ganghöhe der dadurch induzierten cholesterischen Helixstruktur die Temperaturabhängigkeit der elektrooptischen Kennlinien vorteilhaft beeinflußt werden, so beim cholesterisch-nematischen Phasenumwandlungseffekt, der TN("twisted nematic")-Zelle und dem kürzlich vorgestellten SBE ("supertwisted birefringence effect"). Die üblichen bekannten Dotierstoffe induzieren im allgemeinen eine mit zunehmender Temperatur ansteigende Ganghöhe; es sind in jüngster Zeit auch bereits Dotierstoffe beschrieben worden, die diesen oftmals unerwünschten Effekt nicht zeigen.

Aus der DE-C 28 27 471 (= US-A 4 264 148) ist der Zusatz von zwei unterschiedlichen chiralen Dotierstoffen zu nematischen Trägersubstanzen bekannt; dabei erzeugt der eine chirale Dotierstoff in der nematischen Trägersubstanz eine rechtshändige Verdrillung, der andere eine linkshändige Verdrillung. Mit einer solchen Dotierung wird eine Abnahme der Ganghöhe erreicht, aber es sind zur Erreichung dieses Effekts relativ hohe Gesamtkonzentrationen erforderlich, die zu einer negativen Beeinflussung der anderen Materialparameter führen können.

In der DE-A 33 33 677 werden u.a. Umsetzungsprodukte (Ester) von chiralem Butandiol-(2,3) mit mesogenen Carbonsäuren beschrieben, die bereits in Einzeldosierung in Flüssigkristall-Phasen die Optimierung der Temperaturkompensation vereinfachen können. Diese bekannten Ester weisen aber oftmals ein für bestimmte Anwendungen noch zu niedriges Verdrillungsvermögen auf.

Aufgabe der vorliegenden Erfindung ist es deshalb, neue Verbindungen aufzufinden, die bei ihrem Einsatz als chirale Dotierstoffe in Flüssigkristall-Phasen bei verhältnismäßig geringen Zusatzmengen einzeln oder im Gemisch bereits eine Optimierung der Temperaturkompensation und gleichzeitig eine starke Verdrillung der Flüssigkristall-Phasen bewirken; außerdem soll es möglich sein, ausgehend von einer vergleichbaren Grundstruktur, durch kleinere Molekülvariationen das Molekül in seinen Eigenschaften in einer bestimmten Richtung zu verändern.

Die Erfindung geht aus von den bekannten Verbindungen aus einem Molekülbaustein mit zwei Chiralitätszentren und mindestens einem mesogenen Molekülbaustein. Die erfindungsgemäßen Verbindungen sind gekennzeichnet durch die allgemeine Formel (I) eines Butantetraol-Derivates

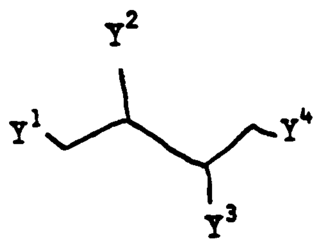

(I)

in der die Symbole folgende Bedeutung haben:
zwei von $Y^1$, $Y^2$, $Y^3$, $Y^4$ sind Hydroxy und MC-CO-O oder unabhängig voneinander MC-CO-O und die anderen zwei unabhängig voneinander ($C_1$-$C_{10}$)Alkoxy, wobei $Y^2$ und $Y^3$ im Falle von ($C_1$-$C_{10}$)Alkoxy gemeinsam auch Teil eines Dioxolan-Rings, oder $Y^1$ und $Y^4$ gemeinsam O als Teil eines Tetrahydrofuran-Rings und $Y^2$ und $Y^3$ Hydroxy und MC-CO-O oder unabhängig voneinander MC-CO-O sein können,
und wobei MC den Molekülrest einer mesogenen Carbonsäure mit der allgemeinen Formel II darstellt,

$R\text{-}(A^1\text{-})_{n1}(B\text{-})_{n2}(A^2\text{-})_{n3}$ (II)

in der die Symbole folgende Bedeutung haben:

| | |
|---|---|
| R ist | ein geradkettiges oder verzweigtes ($C_1$-$C_{12}$)Alkyl, wobei eine oder zwei nicht-benachbarte $CH_2$-Gruppen durch O-Atome ersetzt sein können, oder falls n1 bedeutet 1 auch F, Cl, Br oder CN |
| $A^1$,$A^2$ ist | unabhängig voneinander 1,4-Phenylen, Diazin-2,5-diyl, Diazin-3,6-diyl, 1,4-Cyclohexylen, 1,3-Dioxan-2,5-diyl, 1,3-Dithian-2,5-diyl oder 1,4-Bicyclo(2,2,2)octylen, wobei diese Gruppen auch mindestens einfach substituiert sein können durch F, Cl, Br, CN und/oder ($C_1$-$C_{12}$)Alkyl, in den ein oder zwei nicht-benachbarte $CH_2$-Gruppen durch O-Atome ersetzt |

sein können,

B ist CO-O, O-CO, $CH_2$-$CH_2$, $OCH_2$, $CH_2O$, CH = N, N = CH, N = N, N(O) = N,

n1,n2,n3 ist unabhängig voneinander 0, 1 oder 2, wobei n1 und n3 nicht gleichzeitig 0 sind.

Die genannten Verbindungen sind ein- oder zweifach an den OH-Gruppen veresterte und zweifach veretherte Butan-1,2,3,4-tetraol-Derivate,ein- oder zweifach veresterte 4,5-Dihydroxymethyl-1,3-dioxolan-Derivate oder ein- oder zweifach veresterte 3,4-Dihydroxy-tetrahydrofuran-Derivate.

Eine weitere Lösung der gestellten Aufgabe ist eine verdrillbare Flüssigkristall-Phase mit einem Gehalt an mindestens einer chiralen Verbindung, die dadurch gekennzeichnet ist, daß sie als chirale Verbindung mindestens eine Verbindung der allgemeinen Formel (I) enthält. Unter dem Begriff "verdrillbare Flüssigkristall-Phase" sind nematische, cholesterische, geneigt("tilted")-smektische, insbesondere smektisch C($S_c$ oder SmC), Phasen zu verstehen.

Die erfindungsgemäßen verdrillbaren Flüssigkristallphasen bestehen aus 2 bis 20, vorzugsweise 2 bis 15 Komponenten, darunter mindestens einem der erfindungsgemäß beanspruchten chiralen Dotierstoffe. Die anderen Bestandteile werden vorzugsweise ausgewählt aus den bekannten Verbindungen mit nematischen, cholesterischen und/oder geneigtsmektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe des chiralen Dotierstoffes als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [= mindestens eine enantiotrope (Klärtemperatur > Schmelztemperatur) oder monotrope (Klärtemperatur < Schmelztemperatur) Mesophasenbildung erwarten läßt].

Mit Hilfe der neu-entwickelten Verbindungen als Dotierstoff gelingt es bei geringer Menne an Dotierstoff in Flüssigkristall-Phasen eine hohe Verdrillung zu erzielen, wobei die Verbindungen einzeln oder im Gemisch außerdem eine bei Temperaturänderung im wesentlichen unabhängige Ganghöhe aufweisen können, d.h. die Zu- oder Abnahme der Ganghöhe liegt im allgemeinen im Bereich von 1 % bis 1 ‰ pro K. Ein weiterer Einsatz der erfindungsgemäßen Verbindungen kann bei der Thermotopographie oder zur Erzeugung von "blue phases" (=cholesterische Systeme mit relativ kleiner Ganghöhe von z.B. weniger als 800 nm) erfolgen.

Ein weiterer Gegenstand der Erfindung ist ein Flüssigkristall-Anzeigeelement, enthaltend eine verdrillbare Flüssigkristall-Phase mit einem Gehalt an mindestens einer chiralen Verbindung, der allgemeinen Formel (I).

Die Erfindung beinhaltet zudem ein Verfahren zur Temperaturkompensation und Verdrillung in Flüssigkristall-Anzeigeelementen, die eine Flüssigkristall-Phase enthalten, durch Zusatz von 0,01 bis 70 Gew.-% mindestens einer Verbindung gemäß der allgemeinen Formel (I).

Unter den Verbindungen der Formel I sind die bevorzugt, bei denen die Symbole folgende Bedeutung haben: R = geradkettiges ($C_4$-$C_{10}$)Alkyl, wobei eine $CH_2$-Gruppe durch ein O-Atom ersetzt sein kann, $A^1$,$A^2$ = unabhängig voneinander unsubstituiertes 1,4-Phenylen, Diazin-2,5-diyl oder 1,4-Cyclohexylen, B = CO-O oder O-CO, n1 = 1, n2 = 0 oder 1, und n3 = 1 oder 2.

In der allgemeinen Formel (I) werden darüber hinaus die Verbindungen bevorzugt, bei denen die Symbole folgende Bedeutung haben: a) $Y^1 = Y^4$ = MC-CO-O oder $Y^1$ = Hydroxy und $Y^4$ = MC-CO-O und $Y^2 = Y^3$ = ($C_1$-$C_5$)Alkoxy oder im 1,3-Dioxolan-Ring gemeinsam die Ringstellungen 1 bis 3 mit $C_1$-$C_5$ in Stellung 2, b) $Y^2 = Y^3$ = MC-CO-O oder $Y^2$ = OH und $Y^3$ = MC-CO-O und $Y^1$ und $Y^4$ gemeinsam O als Teil eines Tetrahydrofuran-Rings.

Von der oder den erfindungsgemäßen Dotierstoffen enthalten die Flüssigkristall-Phasen im allgemeinen 0,01 bis 70 Gew.-%, insbesondere 0,05 bis 50 Gew.-%.

## Beispiele

### Allgemeine Arbeitsvorschrift zur Herstellung der Verbindungen 1 bis 5

Zu 1 mmol des R,R-(+)-2,3-Dimethoxy-1,4-butandiols in 10 bis 50 ml wasserfreiem Methylenchlorid oder Dimethylformamid werden unter Rühren 10 bis 40 mg Dimethylaminopyridin und 1,5 mmol der mesogenen Carbonsäure zugegeben. Bei einer Temperatur von 0 °C werden 1,5 mmol Dicyclohexylcarbodiimid hinzugefügt, und es wird während 10 min bei dieser Temperatur und dann 20 h bei Raumtemperatur gerührt. Es wird von ausgefallenem Harnstoff abfiltriert, das Filtrat wird im Vakuum eingedampft und der verbleibende Rückstand wird in Methylenchlorid aufgenommen. Nach eventueller Filtration wird das organische Lösemittel abgezogen und der Rückstand an Kieselgel chromatographiert. Nach dieser Vorschrift

[siehe auch D. Seebach, Helv. Chim. Acta 60, 301 1977)] werden folgende Verbindungen synthetisiert, deren Struktur durch spektroskopische Daten und Elementaranalysen gesichert sind:

(Ausgehend von dem R,R-Derivat)

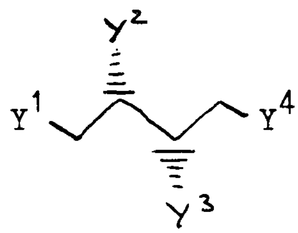

$Y^2 = Y^3$ : $O-CH_3$

(1) $Y^1 = Y^4$ :

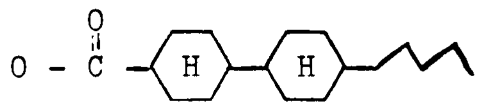

R,R-2,3-Dimethoxy-1,4-bis-(4'-trans-n-pentyl-4-trans-dicyclohexyl-carbonyloxy)-butan

(2) $Y^1 = Y^4$ :

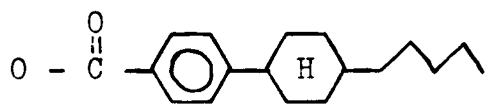

R,R-2,3-Dimethoxy-1,4-bis-[4-(trans-4-n-pentyl-cyclohexyl)-benzoyloxy]-butan

(3) $Y^1 = Y^4$ :

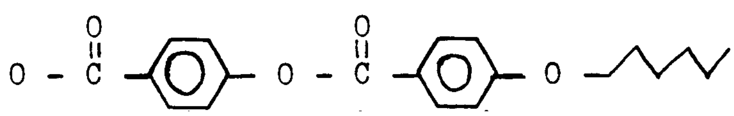

R,R-2,3-Dimethoxy-1,4-bis-[4-(4-n-hexyloxy-benzoyloxy)-benzoyloxy]-butan

(4) $Y^1 = OH$

$Y^4 =$ 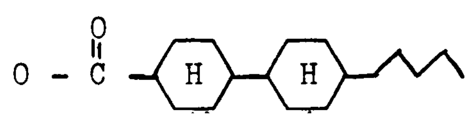

R,R-1-Hydroxy-2,3-dimethoxy-4-(4'-trans-n-pentyl-4-trans-dicyclohexyl-carbonyloxy)-butan

(5) $Y^1 = OH$

$Y^4 =$ 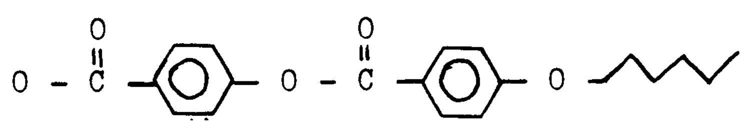

R,R-1-Hydroxy-2,3-dimethoxy-4-[4-(4-n-hexyloxy-benzoyloxy)-benzoyloxy]-butan

Herstellung von Verbindungen 6 bis 8

Es wird nach der vorstehenden allgemeinen Arbeitsvorschrift verfahren, aber ausgehend von dem R,R-(+)-2,2-Dimethyl-4,5-bis(hydroxymethyl)-1,3-dioxolan [siehe A. Halay, Coll. Czech. Chem. Commun. 47, 173

(1982)].

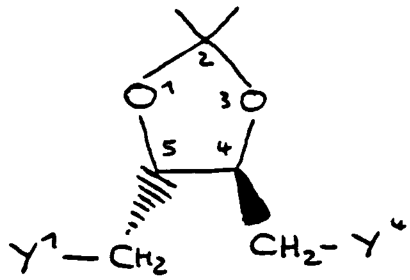

$Y^2 + Y^3$ gemeinsam:

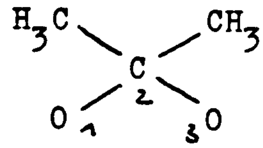

(6) $Y^1 = Y^4$ :

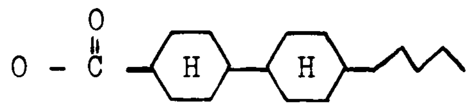

R,R-2,2-Dimethyl-4,5-bis(4'-trans-n-pentyl-4-trans-dicyclohexyl-carbonyloxy-methyl)-1,3-dioxolan

(7) $Y^1 = Y^4$ :

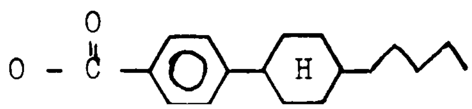

R,R-(-)2,2-Dimethyl-4,5-bis[4-(trans-4-n-pentyl-cyclohexyl)-benzoyloxy-methyl]-1,3-dioxolan

(8) $Y^1 = Y^4$ :

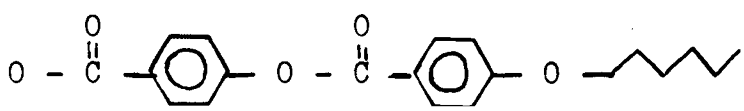

R,R-(-)2,2-Dimethyl-4,5-bis[4-(4-n-hexyloxy-benzoyloxy)-benzoyloxy-methyl]-1,3-dioxolan

Herstellung der Verbindungen 9 und 10

Es wird nach der vorstehend allgemeinen Arbeitsvorschrift verfahren, aber ausgehend von dem R,R-(+)-3,4-Dihydroxy-tetrahydrofuran (siehe F.C. Hartmen und R. Barker, J. Org. Chem. 28, 1004 (1963)].

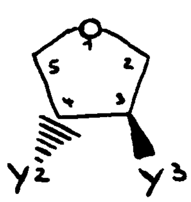

$Y^1 + Y^4$ gemeinsam O

(9) $Y^2 = Y^3$ :

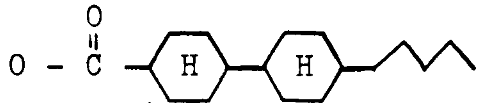

R,R-3,4-Bis(4'-trans-n-pentyl-4-trans-dicyclohexyl-carbonyloxy)-tetrahydrofuran
(10) $Y^2$ = OH

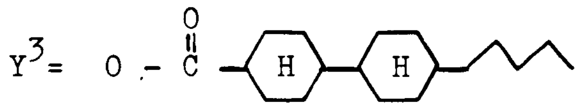

R,R-4-Hydroxy-3-(4'-trans-n-pentyl-4-trans-dicyclohexyl-carbonyloxy)-tetrahydrofuran

Tabelle

| Verbindungs-Nr. | Temperaturintervall der Messung des Verdrillungsvermögens (°C) | Verdrillungsvermögen p•c (µm•Gew.-%) | Schmelzpunkt (°C) |
|---|---|---|---|
| 1 | 10 - 100 | -16 → 12 | 103-111 |
| 2 | 10 - 100 | -12 → - 9 | 109-110 |
| 3 | 10 - 100 | -13 → -10,5 | 118-119 |
| 4 | - | - | 192-194 |
| 5 | - | - | 56- 58 |
| 6 | 10 - 100 | -16 → -47 | 94-96 |
| 7 | - | - | 148-150 |
| 8 | - | - | - |
| 9 | - | - | 185-187 |
| 10 | - | - | 234 |

Die Messung des Verdrillungsvermögens wird in einer handelsüblichen nematischen Weitbereichsmischung -"RO-TN 404" der Hoffmann-La Roche Aktiengesellschaft (Basel/Schweiz)-mit einem Klärpunkt von 104 ° C durchgeführt.

In der anliegenden Zeichnung sind die HTP-Werte in Abhängigkeit von der Temperatur für verschiedene der erfindungsgemäßen Verbindungen aufgetragen.

- HTP ("helical twisting power") = 1/p•c (p-Ganghöhe der induzierten Helixstruktur in µm, c = Konzentration des chiralen Dotierstoffes in Gew.-%)

Vergleicht man in der Zeichung den Kurvenverlauf der HTP-Werte der Verbindungen 1 und 6 miteinander, so sieht man, daß das R,R-Derivat 1 eine linkshändige Helix mit steigender HTP bei zunehmender Temperatur induziert, verknüpft man jedoch die Substituenten $Y^2$ und $Y^3$ zu einem Teil eines Dioxolanrings, so fällt die HTP bei zunehmender Temperatur.

## Patentansprüche

1. Verbindungen aus einem Molekülbaustein mit zwei Chiralitätszentren und mindestens einem mesogenen Molekülbaustein gekennzeichnet durch die allgemeine Formel (I),

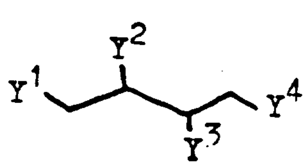

(I)

in der die Symbole folgende Bedeutung haben:
zwei von $Y^1$, $Y^2$, $Y^3$, $Y^4$ sind Hydroxy und MC-CO-O oder unabhängig voneinander MC-CO-O und die anderen zwei unabhängig voneinander ($C_1$-$C_{10}$)Alkoxy, wobei $Y^2$ und $Y^3$ im Falle von ($C_1$-$C_{10}$)Alkoxy gemeinsam auch Teil eines Dioxolan-Rings, oder $Y^1$ und $Y^4$ gemeinsam O als Teil eines Tetrahydrofuran-Rings und $Y^2$ und $Y^3$ Hydroxy und MC-CO-O oder unabhängig voneinander MC-CO-O sein können,
und wobei MC den Molekülrest einer mesogenen Carbonsäure mit der allgemeinen Formel II darstellt,

$R\text{-}(A^1\text{-})_{n1}(B\text{-})_{n2}(A^2\text{-})_{n3}$ (II)

in der die Symbole folgende Bedeutung haben:

| | |
|---|---|
| R ist | ein geradkettiges oder verzweigtes ($C_1$-$C_{12}$)Alkyl, wobei eine oder zwei nicht-benachbarte $CH_2$-Gruppen durch O-Atome ersetzt sein können, oder falls n1 bedeutet 1 auch F, Cl, Br oder CN |
| $A^1$,$A^2$ ist | unabhängig voneinander 1,4-Phenylen, Diazin-2,5-diyl, Diazin-3,6-diyl, 1,4-Cyclohexylen, 1,3-Dioxan-2,5-diyl, 1,3-Dithian-2,5-diyl oder 1,4-Bicyclo(2,2,2)octylen, wobei diese Gruppen auch mindestens einfach substituiert sein können durch F, Cl, Br, CN und/oder ($C_1$-$C_{12}$)Alkyl, in den ein oder zwei nicht-benachbarte $CH_2$-Gruppen durch O-Atome ersetzt sein können, |
| B ist | CO-O, O-CO, $CH_2$-$CH_2$, $OCH_2$, $CH_2O$, CH = N, N = CH, N = N, N(O) = N, |
| n1,n2,n3 ist | unabhängig voneinander 0, 1 oder 2, wobei n1 und n3 nicht gleichzeitig 0 sind. |

**2.** Verdrillbare Flüssigkristall-Phase mit einem Gehalt an mindestens einer chiralen Verbindung, dadurch gekennzeichnet, daß sie mindestens eine chirale Verbindung gemäß der allgemeinen Formel (I) nach Anspruch 1 enthält.

**3.** Flüssigkristall-Phase nach Anspruch 2, dadurch gekennzeichnet, daß sie 0,01 bis 70 Gew.-% an mindestens einer der chiralen Verbindungen enthält.

**4.** Flüssigkristall-Anzeigeelement enthaltend eine Flüssigkristall-Phase nach Anspruch 2.

**5.** Verwendung einer chiralen Verbindung gemäß der allgemeinen Formel (I) nach Anspruch 1 zur Temperaturkompensation und Erzeugung einer Verdrillung in Flüssigkristall-Phasen.

**6.** Verfahren zur Temperaturkompensation und Verdrillung in Flüssigkristall-Anzeigeelementen, die eine Flüssigkristall-Phase enthalten, durch Zusatz von mindestens einer chiralen Verbindung, dadurch gekennzeichnet, daß man der Flüssigkristall-Phase 0,01 bis 70 Gew.-% mindestens eine Verbindung gemäß der allgemeinen Formel (I) nach Anspruch 1 zusetzt.

## Claims

**1.** A compound comprising a molecular structural element with two chirality centers and at least one mesogenic molecular structural element which is defined by the formula (I)

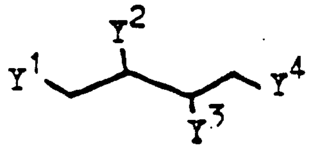

(I)

in which the symbols have the following meanings:
two of $Y^1$, $Y^2$, $Y^3$, $Y^4$ are hydroxyl and MC-CO-O, or independently of each other MC-CO-O and the other two, independently of each other, are ($C_1$-$C_{10}$)alkoxy, it being possible for $Y^2$ and $Y^3$, in the case of ($C_1$-$C_{10}$)alkoxy, also to be jointly part of a dioxolane ring, or
for $Y^1$ and $Y^4$ jointly to be O as part of a tetrahydrofuran ring, and for $Y^2$ and $Y^3$ to be hydroxyl and MC-CO-O or, independently of each other, MC-CO-O,

and MC being the molecular residue of mesogenic carboxylic acid having the formula (II)

$R\text{-}(A^1\text{-})_{n1}(B\text{-})_{n2}(A^2\text{-})_{n3}$ (II)

in which the symbols have the following meanings:

| | |
|---|---|
| R | is a straight-chain or branched ($C_1$-$C_{12}$) alkyl, it being possible for one or two non-adjacent $CH_2$ groups to be replaced by O atoms, or if n1 is 1 also F, Cl, Br or CN, |
| $A^1$, $A^2$ | are independently of each other, 1,4-phenylene, diazine-2,5-diyl, diazine-3,6-diyl, 1,4-cyclohexylene, 1,3-dioxane-2,5-diyl, 1,3-dithiane-2,5-diyl or 1,4-bicyclo-[2.2.2]octylene, it being possible for these groups also to be substituted at least singly by F, Cl, Br, CN and/or ($C_1$-$C_{12}$)alkyl in which one or two non-adjacent $CH_2$ groups may be replaced by O atoms, |
| B | is CO-O, O-CO, $CH_2$-$CH_2$, $OCH_2$, $CH_2O$, CH = N, N = CH, N = N, N(O) = N, |
| n1, n2, n3 | are independently of each other, 0, 1 or 2, n1 and n3 not being 0 at the same time. |

**2.** A twistable liquid crystal phase containing at least one chiral compound, wherein the phase contains at least one chiral compound of the formula (I) as claimed in claim 1.

**3.** A liquid crystal phase as claimed in claim 2, wherein the phase contains 0.01 to 70% by weight of at least one of the chiral compounds.

**4.** A liquid crystal display element containing a liquid crystal phase as claimed in claim 2.

**5.** The use of a chiral compound according to the formula (I) as claimed in claim 1 for temperature compensation and producing twisting in liquid crystal phases.

**6.** A method for temperature compensation and twisting in liquid crystal display elements, which contain a liquid crystal phase, by adding at least one chiral compound, wherein 0.01 to 70% by weight of at least one compound according to formula (I) as claimed in claim 1 is added to the liquid crystal phase.

## Revendications

**1.** Composés formés d'un élément moléculaire à deux centres de chiralité et d'un ou de plusieurs éléments moléculaires mésogènes, caractérisés par la formule générale I ci-dessous :

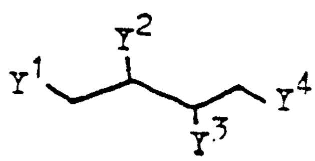

(I)

dans laquelle les divers symboles ont les significations suivantes :
parmi $Y^1$, $Y^2$, $Y^3$ et $Y^4$ deux sont un hydroxyle et un groupe MC-CO-O ou bien, indépendamment l'un de l'autre, un groupe MC-CO-O, et les deux autres, indépendamment l'un de l'autre, un alcoxy en $C_1$-$C_{10}$, $Y^2$ et $Y^3$, dans le cas d'alcoxy en $C_1$-$C_{10}$, pouvant aussi faire partie ensemble d'un cycle de dioxolanne, ou bien $Y^1$ et $Y^4$ pouvant avec O faire partie d'un cycle de tétrahydrofuranne et $Y^2$ et $Y^3$ pouvant être un hydroxyle et un groupe MC-CO-O ou encore, indépendamment l'un de l'autre, un groupe MC-CO-O,
et MC représente le radical moléculaire d'un acide carboxylique mésogène de formule générale II :

$R\text{-}(A^1\text{-})_{n1}(B\text{-})_{n2}(A^2\text{-})_{n3}$ (II)

dans laquelle les divers symboles ont les significations suivantes :

| | |
|---|---|
| R | est un alkyle linéaire ou ramifié en $C_1$-$C_{12}$ dont un ou deux groupes $CH_2$ non voisins peuvent être remplacés par des atomes d'oxygène, ou bien, si n1 = 1, également F, Cl, Br ou CN, |
| $A^1$ et $A^2$ | sont, indépendamment l'un de l'autre, un groupe 1,4-phénylène, diazine-2,5-diyle, |

diazine-3,6-diyle, 1,4-cyclohexylène, 1,3-dioxanne-2,5-diyle, 1,3-dithianne-2,5-diyle ou 1,4-bicyclo(2,2,2)octylène, groupes qui peuvent aussi comporter un ou plusieurs substituants pris parmi F, Cl, Br, CN et/ou des alkyles en $C_1$-$C_{12}$ dont un ou deux groupes $CH_2$ non voisins peuvent être remplacés par des atomes d'oxygène,

B est un groupe CO-O, O-CO, $CH_2$-$CH_2$, $OCH_2$, $CH_2O$,CH=N, N=CH, N=N ou N(O)-=N et

n1, n2 et n3 sont chacun, indépendamment les uns des autres, le nombre 0, 1 ou 2, mais n1 et n3 ne sont pas nuls à la fois.

**2.** Phase de cristaux liquides torsadable comprenant un ou plusieurs composés chiraux, caractérisée en ce qu'elle contient un ou plusieurs composés chiraux de la formule I de la revendication 1.

**3.** Phase de cristaux liquides selon la revendication 2, caractérisée en ce qu'elle comprend de 0,01 à 70 % en poids d'un ou de plusieurs des composés chiraux spécifiés.

**4.** Elément d'affichage à cristaux liquides contenant une phase de cristaux liquides selon la revendication 2.

**5.** Emploi d'un composé chiral de la formule générale I de la revendication 1 pour une compensation de température et produire une torsion dans des phases de cristaux liquides.

**6.** Procédé de compensation de température et de torsion dans des éléments d'affichage à cristaux liquides contenant une phase de cristaux liquides, par l'addition d'un ou de plusieurs composés chiraux, procédé caractérisé en ce que l'on ajoute à la phase de cristaux liquides de 0,01 à 70 % en poids d'un ou de plusieurs composés de la formule générale I de la revendication 1.

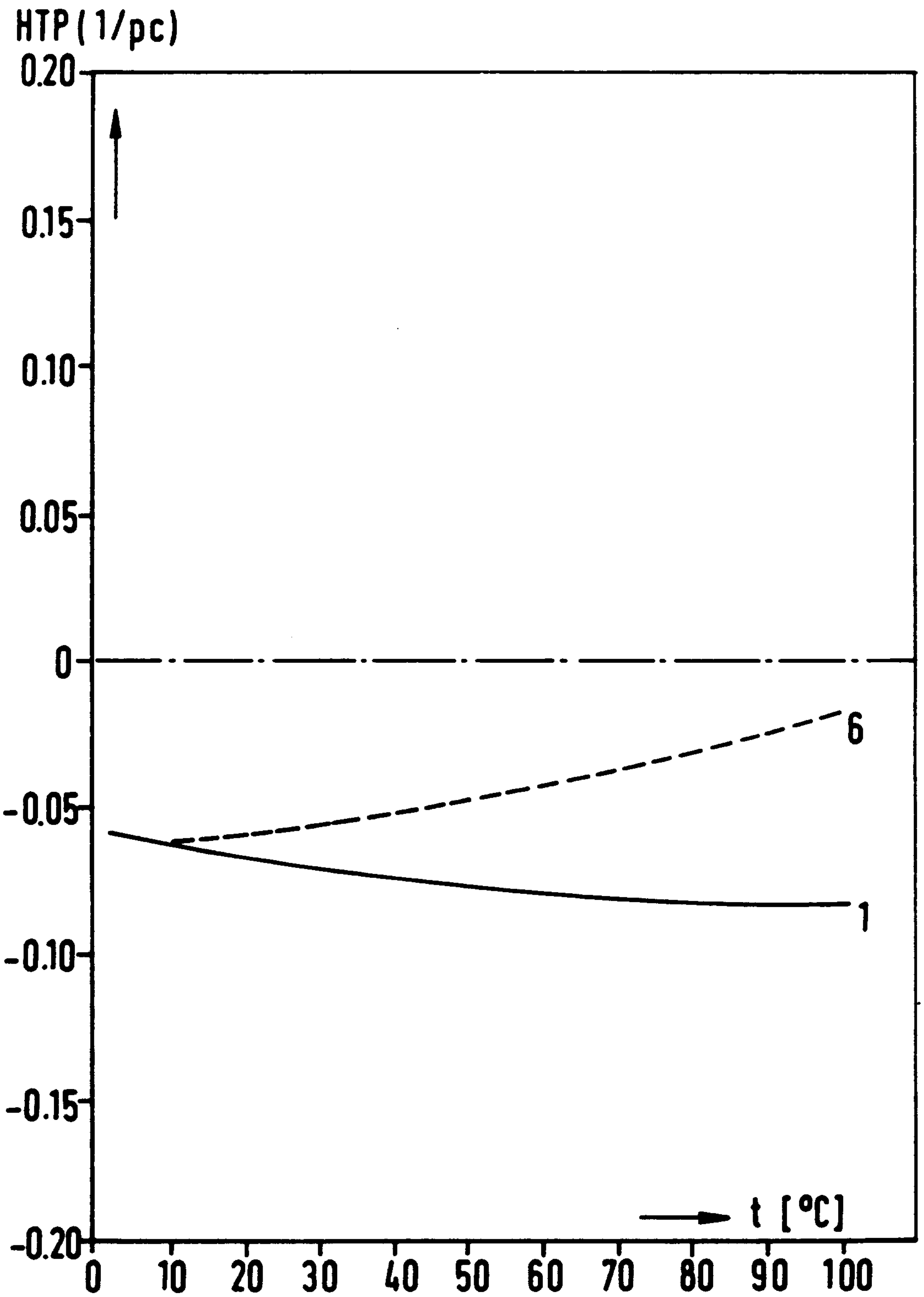
HTP (1/pc)
0.20
0.15
0.10
0.05
0
-0.05
-0.10
-0.15
-0.20
6
1
t [°C]
0
10
20
30
40
50
60
70
80
90
100